Europäisches Patentamt

European Patent Office

Office européen des brevets

(19)

(11) Publication number: **0 431 844 A2**

## EUROPEAN PATENT APPLICATION

(12)

(21) Application number: 90313024.3

(22) Date of filing: 30.11.90

(51) Int. Cl.⁵: **A01N 25/18,** A01N 59/02, A61L 2/20

(30) Priority: 04.12.89 GB 8927386

(43) Date of publication of application: 12.06.91 Bulletin 91/24

(84) Designated Contracting States: BE CH DE DK ES FR GB IT LI NL SE

(71) Applicant: RENTOKIL LIMITED Felcourt East Grinstead, West Sussex RH19 2JY (GB)

(72) Inventor: Lawson, Trevor John Corner Cottage, The Limes, Felbridge West Sussex, RH19 2QY (GB) Inventor: Lockyer, Martin Hewitt 118 Hopgarden Road, Tonbridge Kent, TN10 4QX (GB)

(74) Representative: Carter, Caroline Ann et al ABEL & IMRAY Northumberland House 303-306 High Holborn London WC1V 7LH (GB)

(54) Sterilizing compositions and their use.

(57)     A composition and preparation capable of giving extended release of an airborne disinfectant, particularly sulphur dioxide, in the presence of moisture. The preparation, which is conveniently supplied in a sealed container which, when opened, can be installed in a disposal unit for sanitary or surgical dressings to sterilise the contents, preferably comprises an agent that will liberate sulphur dioxide on contact with water, · a moderator for moderating the liberation of sulphur dioxide, a thickening agent, and water, the preparation being in the form of a stable viscous solution.

EP 0 431 844 A2

## STERILIZING COMPOSITIONS AND THEIR USE

This invention relates to compositions capable of giving extended release of airborne disinfectants, particularly sulphur dioxide. Those compositions are particularly useful, _inter alia_, for sterilizing articles, and are especially suitable for use in the temporary disposal and storage of soiled sanitary and surgical dressings under sterile conditions prior to permanent disposal of the dressings, for example, by incineration.

Soiled dressings are an extremely efficient bacterial growth medium and various methods have been proposed for their temporary disposal. Such temporary disposal can be problematic, however, especially in such situations as public conveniences where a disposal unit for sanitary dressings may not be emptied for a period of several days or even weeks.

Sulphur dioxide has been known for many years to be a very efficient sterilizing agent, both in solution and in vapour form in a moist atmosphere. It has been found to be an excellent sterilizing medium for articles such, for example, as soiled sanitary and surgical dressings, in that it slows bacterial growth very considerably or even eliminates it. It has the additional advantage that, because of its reducing action, it has an inhibiting effect on oxidation reactions, which frequently produce malodours.

One problem associated with the use of sulphur dioxide for such purposes as the temporary disposal of soiled dressings is that its sterilizing action is transitory. This is because most chemical reactions producing sulphur dioxide are fast reactions with the gaseous sulphur dioxide being evolved fairly quickly so that the system is exhausted after a fairly short period of time. The sulphur dioxide produced gradually diffuses into the atmosphere and leaves a sulphur dioxide concentration that is insufficient to be effective.

A solution to the above problem is described in British Patent Specification No. 1 531 722 in the name of Rentokil Limited. That specification describes and claims a composition capable of giving extended release of sulphur dioxide in the presence of moisture, which comprises a thiosulphate and a compound that will liberate sulphur dioxide on contact with water. The thiosulphate acts as a sulphur dioxide-liberating moderator so that the composition is not rapidly exhausted but continues to liberate sulphur dioxide for a prolonged period of time. By using such a composition in a closed, but periodically opened, container such as a disposal unit for sanitary dressings, it is possible to maintain an active concentration of sulphur dioxide within the container for a period of up to several weeks.

In general, the charging of a disposal unit with the above-described composition capable of giving extended release of sulphur dioxide involves dissolving the desired quantity of the composition in, say, a litre of water in the bottom of the disposal unit, the composition being introduced into the unit in powder form, in a water-soluble sachet. This method, and the aqueous solution obtained by the method, have proved very satisfactory in use. When, however, the disposal unit is emptied, there is a relatively large quantity of liquid having a relatively high salt concentration to be disposed of, which may be disadvantageous in some circumstances.

The present invention provides a composition which comprises a thickening agent and an agent capable of liberating a vapour-phase disinfectant on contact with water, the proportion by weight and nature of the thickening agent being such that, when the composition is mixed with the minimum amount of water required to dissolve the said agent, a stable viscous solution (as hereinafter defined) is formed. The composition is preferably capable of giving extended release of the vapour-phase disinfectant in the presence of moisture.

The term "vapour-phase disinfectant" is intended to include all airborne disinfectants. Thus, for example, the term includes both gases and vapours, and, in particular, includes sulphur dioxide in gaseous or vapour form.

The present invention also provides a composition capable of giving extended release of sulphur dioxide in the presence of moisture, which comprises an agent that will liberate sulphur dioxide on contact with water, a moderator for moderating the liberation of sulphur dioxide, and a thickening agent, the proportion by weight and nature of the thickening agent being such that, when the composition is mixed with the minimum amount of water required to dissolve the sulphur dioxide-liberating agent and the moderator, a stable viscous solution (as hereinafter defined) is formed.

By a viscous solution is meant a solution having a viscosity greater than that of water at the same temperature, and preferably at least 10 Pa.s. Advantageously, the viscosity is at least 20 Pa.s. Provided that the vapour-phase disinfectant can still be released at effective levels, there is no upper limit on the viscosity, but for ease of processing the viscosity of the viscous solution preferably does not exceed 200 Pa.s immediately after preparation of the solution. Advantageously the viscosity, immediately after preparation of the viscous solution, is in the range of 10 Pa.s to 100 Pa.s, preferably 20 Pa.s to 40 Pa.s.

A viscous solution is said to be stable if, on exposure, at 20°C, of the solution to air, it retains a viscosity greater than that of water at that temperature, advantageously at least 10 Pa.s, and preferably at least 20 Pa.s, for a period of at least two weeks, advantageously at least four weeks, and, preferably, at least six weeks. A

2

"stable viscous solution" as defined herein is thus a solution having a viscosity greater than that of water at the same temperature, preferably at least 10 Pa.s, and advantageously at least 20 Pa.s, and which, on exposure, at 20°C, to air, retains a viscosity greater than that of water at that temperature, preferably greater than 10 Pa.s, and advantageously at least 20 Pa.s, for at least two weeks.

All viscosities given herein are as measured using a Brookfield LVT viscometer at 25°C using Spindle No. 3 rotating at 6 rpm.

The invention further provides a preparation which comprises a thickening agent and an agent capable of liberating a vapour-phase disinfectant on contact with water, the preparation being in the form of a stable viscous solution (as hereinbefore defined). The preparation is preferably capable of giving extended release of the vapour-phase disinfectant.

The invention also provides a preparation capable of giving extended release of sulphur dioxide, which comprises an agent that will liberate sulphur dioxide on contact with water, a moderator for moderating the liberation of sulphur dioxide, a thickening agent, and water, the preparation being in the form of a stable viscous solution (as hereinbefore defined).

As the preferred vapour-phase disinfectant is sulphur dioxide, the invention will hereinafter be described with reference to that compound. It is however to be understood that, unless this is clearly inappropriate in a given context, sulphur dioxide could be replaced by another vapour-phase disinfectant, and a sulphur-dioxide-liberating agent/moderator system could be replaced by an agent or system capable of liberating another vapour-phase disinfectant.

As the preparation of the invention contains water it will, in an open system, liberate sulphur dioxide, and other changes may (see below) take place. If however the preparation is stored in a closed container, release of sulphur dioxide and changes possibly associated therewith are substantially prevented. The invention also provides a closed container containing the preparation of the invention or an aqueous solution of the composition of the invention. Advantageously, such a container is provided with a removable closure member, for example, a lid that can be peeled off when the preparation or composition is to be used.

Compounds that will liberate sulphur dioxide in the presence of water are well known. For example, metabisulphites and dithionites will decompose in the presence of water with the liberation of sulphur dioxide, and these compounds have been found to be particularly suitable for use as sulphur dioxide-liberating agents in accordance with the invention. They are conveniently used in the form of the respective sodium salts ($Na_2S_2O_5$ and $Na_2S_2O_4$ respectively) since these are readily obtainable.

Various moderators may be used in accordance with the invention, and the particular moderator chosen in any given case will depend partly on the sulphur dioxide-liberating agent used and also on the intended use. In general terms, the effect of the moderator is to decrease the rate at which the sulphur dioxide is liberated from the sulphur dioxide-liberating agent, as compared with the rate at which it is liberated in the absence of the moderator. That is not to say that the presence of the moderator results in the liberation of substantially less sulphur dioxide, but that it results in the liberation of a substantially similar amount of sulphur dioxide, as compared with the amount liberated in the absence of the moderator, at a lower rate and thus over an extended period of time. Thus, the composition or preparation is described as being "capable of giving extended release of sulphur dioxide".

While all the moderators have the above-described general effect, this can be manifested in different ways by different moderators. Certain moderators, for example, will result in the liberation of sulphur dioxide at a substantially constant rate over a period of several days or even weeks, and this can be particularly advantageous for use in such situations as disposal units for sanitary dressings in public conveniences as described above. Other moderators will result in the liberation of sulphur dioxide at a gradually decreasing rate, and this can be useful in situations where it is desired to establish fairly rapidly an active concentration of sulphur dioxide and then to maintain an active concentration over a period of time by the continued liberation of sulphur dioxide at a decreased rate to compensate for leakage of the sulphur dioxide. Still other moderators will result in the liberation of sulphur dioxide at an initially increasing rate and then at a decreasing rate, and this can be useful where it is desired to effect a gradual build-up of the concentration of sulphur dioxide and then to maintain an active concentration. In all cases, however, the sulphur dioxide is liberated over a period of time greater than that over which it would be liberated in the absence of a moderator.

The action of a particular moderator, and thus its suitability for a particular purpose, may be ascertained by means of simple preliminary tests, such as those described below in Example 1.

The manner in which the moderator functions to cause the slower liberation of the sulphur dioxide is not clear or certain, but again appears to vary according to the particular moderator used. Certain moderators, for example, are simply buffers and appear to function by maintaining the pH of an aqueous solution of the composition at a value such that sulphur dioxide is liberated at a decreased rate. The manner in which other moderators function appears to be somewhat more complicated. It is thought that some moderators function by taking up a certain proportion of the sulphur dioxide given off by the sulphur dioxide-liberating agent and returning this proportion to the sulphur dioxide-liberating agent, so that only a certain amount of free sulphur dioxide is liberated in each cycle of reactions. In any case the moderator of the invention is generally a "chemical" moderator, that is, the chemical nature or effect of the moderator is important, and the moderator generally does not operate purely as a result of physical factors. (It is to be emphasised, however, that the present invention is not restricted to any particular mode of action of the moderator or to any particular chemical theory as to the manner in which the moderator functions.) For certain applications, it can be advantageous to use a combination of two moderators, one of which functions in one manner and the other of which functions in another manner.

Thiosulphates have been found to be particularly effective as moderators in accordance with the invention, the sodium salt ($Na_2S_2O_3$) generally being used since it is readily available. A thiosulphate may be used as the sole moderator, but in many cases it is advantageous to use a thiosulphate in conjunction with a second moderator that is a buffer.

Certain phosphates have also proved to be particularly effective as moderators, both as the sole moderator and, especially, in conjunction with a thiosulphate. Orthophosphates are especially to be mentioned in this respect, for example potassium dihydrogen orthophosphate ($KH_2PO_4$). Examples of other buffers that are suitable for use as moderators are borates, for example, a boric acid-borate mixture or borax, and amines, for example, triethylamine. Hydroquinones may also be used as moderators.

In the case of moderators that serve as buffers, the pH at which an aqueous solution is buffered depends both on the particular sulphur dioxide-liberating compound being used and on the desired rate of liberation of sulphur dioxide, as well as on the chosen moderator. The optimum pH for a particular use may be determined as mentioned above by means of simple preliminary tests.

The relative proportions of sulphur dioxide-liberating compound and moderator may vary within wide limits: in some cases, the sulphur dioxide-liberating compound may be present in the greater amount by weight while in other cases the moderator may be present in the greater amount. Again, the proportions chosen depend on the intended use but may be determined by simple tests.

Especially preferred compositions and preparations according to the invention comprise a metabisulphite as the sulphur dioxide-liberating agent and comprise both a thiosulphate and a phosphate as moderators. Examples of other compositions and preparations according to the invention comprising a metabisulphite as the sulphur dioxide-liberating agent are those comprising, as the moderator, a thiosulphate, a phosphate, an amine, or a hydroquinone. Examples of compositions and preparations comprising a dithionite as the sulphur dioxide-liberating agent are those comprising, as the moderator, a thiosulphate, a phosphate, a hydroquinone, or a borate.

The proportion by weight and nature of the thickening agent must be such that a stable viscous solution (as hereinbefore defined) is formed when the sulphur dioxide-liberating compound, the moderator, and the thickening agent are dissolved in the minimum amount of water required to dissolve the sulphur dioxide-liberating agent and the moderator. When the preparation of the invention is used to provide controlled release of sulphur dioxide it is, if desired, possible to use more than this minimum amount of water, but for economic reasons an amount of water as close as possible to this minimum amount will generally be preferred.

In order to give a stable viscous solution, the thickening agent must be such that it can remain dissolved at the pH and ionic concentrations resulting from the addition to the composition of the invention of the minimum amount of water required to dissolve the sulphur dioxide-liberating agent and the moderator. Thus, for example, the thickening agent preferably does not precipitate out of an aqueous solution with a thiosulphate concentration of 100 g/l and a sulphite concentration of 500 g/l, and a pH of from 2 to 7, for example, about 4. Advantageously, the thickening agent does not precipitate out of an aqueous solution having an ionic concentration of up to 800 g/l.

A preparation in accordance with the invention advantageously retains water for at least the minimum period for which sulphur dioxide is to be liberated. Although in some cases sulphur dioxide may be released in the presence of a moist atmosphere, more reliable control of sulphur dioxide release may be obtained when the sulphur dioxide-liberating compound and moderator are dissolved in water. Thus the preparation preferably retains for at least two weeks, preferably for at least four weeks, sufficient water to maintain the sulphur dioxide-liberating compound and the moderator in solution.

For economic reasons, the thickening agent is preferably such that a viscosity of at least 10 Pa.s can be obtained with a low concentration of thickening agent, for example, a concentration of at most 4% by weight, preferably at most 2% by weight, calculated on the weight of the water. Further, for ease of processing the thickening agent is preferably such that a preparation in accordance with the invention exhibits pseudoplastic properties (low viscosity at high shear and high viscosity at low shear).

The suitability of a thickening agent for use with a particular sulphur dioxide-liberating agent/moderator combination may be determined by simple preliminary tests, for example, as in Example 2 below.

Any thickening agent capable of giving a stable viscous solution as defined above may be used in accordance with the invention. Preferred thickening agents are carbohydrates of high molecular weight (particularly carbohydrate polymers of high molecular weight comprising acidic and/or neutral monosaccharide units joined by glycosidic bonds, including high molecular weight polysaccharides produced by bacterial fermentation) that are capable of giving a stable viscous solution (as hereinbefore defined).

Particularly preferred thickening agents are natural gums and mucilages obtained from plants, and bacterial fermentation products, that are capable of giving a stable viscous solution (as hereinbefore defined). A number of the more common natural gums and mucilages are described in Van Nostrand Reinhold Encyclopedia of Chemistry, Fourth Edition, 1984, which also describes xanthan gums, very high molecular weight polysaccharides produced by pure culture fermentation of glucose by Xanthamonas campestris. Xanthan gums are particularly preferred thickening agents for use in accordance with the invention. Further information regarding xanthan gums may be found, inter alia, in Technical Bulletin DB-15 from Kelco International Limited, 22 Henrietta Street, London WC2E 8NB. Preferred thickening agents are those sold by Kelco under the names "Kelzan", "Rhamsam Gum", and "Welan Gum". "Kelzan" is a trade mark ; both Kelzan industrial grade and Kelzan S dispersible grade are suitable for use in accordance with the invention.

While the preparations of the invention are contained in closed container, they will normally have a viscosity substantially the same as that obtained immediately after the mixing of the constituents. Once the container is opened, however, and release of sulphur dioxide occurs, preparations containing certain of the preferred thickening agents indicated above may increase in viscosity until the preparation is no longer free-flowing. An increase of viscosity of this kind is not objectionable (provided that release of sulphur dioxide from the preparation is still possible) and, from the point of view of handling, may even be advantageous.

A preparation according to the invention is preferably made by dispersing the thickening agent thoroughly in the desired quantity of water, if necessary using a high shear mixer, and then adding to the viscous solution so obtained a mixture of the sulphur-dioxide-liberating compound and the moderator, the preparation being stirred until a homogeneous viscous solution is obtained.

The present invention also provides a method for sterilizing an article, especially a soiled sanitary dressing, a soiled surgical dressing, or a disposable diaper, which comprises bringing the article into contact with or into the proximity of an aqueous composition or a preparation according to the invention. The invention further provides the use of an aqueous composition or preparation according to the invention for sterilizing an article.

The term "sterilize" as used herein means to cause a substantial reduction in the growth of micro-organisms but is not intended to imply that complete sterility is imparted to the article in the sense that all micro-organisms are completely destroyed.

By "bringing the article into the proximity of the composition or preparation", is meant that the article need not be brought into actual contact with the composition or preparation but is brought sufficiently near thereto to be sterilised by sulphur dioxide being liberated from the composition or preparation. It follows that the article must be brought into the region in which there is an active concentration of sulphur dioxide. The article may, for example, be put into a disposal unit for sanitary dressings or other vessel containing a quantity of a composition or preparation according to the invention, and the invention also provides such a disposal unit. Preferably, the composition or preparation is contained in a container which is positioned, for example, hung, in the disposal unit.

In order for the sulphur dioxide to impart sterility to an article, the concentration of sulphur dioxide in the region of the article should generally be at least 10 p.p.m. (parts per million), advantageously at least 20 p.p.m., although the concentration required in any particular situation will depend on the ambient temperature. The term "active concentration" simply means a concentration sufficient to effect some sterilization of the article. Advantageously, the concentration of sulphur dioxide in the region of the article should be within the range of from 20 to 200 p.p.m., particularly 40 to 200 p.p.m.. Preferably a concentration of at least 10 ppm is maintained for a period of at least two weeks, advantageously at least four weeks, and more especially at least six weeks.

The method of the invention is advantageously carried out in a closed receptacle as this hinders or prevents the diffusion of the sulphur dioxide into the atmosphere and thus more readily enables an active concentration of sulphur dioxide to be maintained. It also has the advantage that the operator or user is not subjected to the unpleasantness of the presence of sulphur dioxide or at least that such unpleasantness is minimised.

The compositions, preparations, and method of the invention are particularly suitable for use in closed receptacles of the so-called "trap-top" type, that is to say receptacles having a pivoted top which when opened presents a shelf on which the article to be placed in the receptacle can be placed while keeping the receptacle sealed and which allows the article to drop into the receptacle when the lid is closed. Such receptacles remain sealed at all times, and thus leakage of sulphur dioxide is reduced to a minimum. Receptacles of this type are particularly suitable for use as disposal units for soiled sanitary and surgical dressings. They can be charged with a quantity of a composition or preparation according to the invention, the composition or preparation preferably being in a container, and can then be put into use for up to several weeks, for example three to four weeks, during which time soiled dressings may be placed in the receptacle as required. When full, or after a set interval of time, the receptacle may be replaced by a freshly-charged receptable and then be taken to a disposal site where it can be emptied and the contents destroyed, for example by incineration.

It is possible, using the compositions or preparations according to the invention, to maintain a sulphur dioxide concentration of, for example, from 40 to 200 p.p.m. within such receptacles for a period of three to four weeks and in some cases for six or more weeks. A concentration of this magnitude is generally sufficient to maintain all soiled dressings or other articles within the receptacle in a sterile state until their permanent disposal. This has been found to be the optimum sulphur dioxide concentration range, although concentrations outside this range may be used in certain cases. The concentration should not, however, generally be less than 10 p.p.m., since there is then a danger of its being ineffective, or more than 500 p.p.m., since there is then a danger of unpleasantness to persons in the vicinity of the container.

As indicated above, the composition or preparation is conveniently contained in a container which can be positioned in the disposal unit. In a preferred embodiment of the invention, the preparation may be provided in a sealed container of, for example, plastics material from which a cover may be removed immediately before positioning of the container in a disposal unit. Thus, for example, the container may have a flexible cover which may readily be peeled off. Such containers may, if desired, be filled and sealed in the factory, so that the only actions required of the operator in the field are the removal of the lid and the positioning of the container in the disposal unit. Further, when a unit is to be emptied and re-charged the container may readily be removed from the unit and replaced by a fresh one.

If desired, small amounts of perfume can be included in the compositions and preparation of the invention in order to mask any sulphur dioxide odour. Various other auxiliaries can also be included, for example, auxiliaries to improve the free-flowing properties of individual components of the compositions.

The invention makes it possible to provide a composition and preparation capable of giving release of an airborne disinfectant over a prolonged period of time while eliminating the need for the disposal in the drains of relatively large quantities of liquid having a high salt concentration. Thus, after use the composition or preparation of the invention may be disposed of by landfill or incineration, this being particularly easy when the spent preparation is contained in a container of the type described above. Furthermore, the use of a preparation in the form of a viscous solution facilitates filling of individual containers of the preparation, their installation in disposal units, and subsequent handling.

In the following Examples, Example 1 gives a test for moderators for moderating the liberation of sulphur dioxide, while Examples 2 to 6 illustrate the invention.

Details of substances indicated in the Examples by trade names are as follows :

| | |
|---|---|
| KELZAN : | Xanthan gum (Industrial Grade) - a high-molecular weight branched polysaccharide supplied by Kelco International Limited, and produced by microbial fermentation. |
| RHAMSAN GUM : | A microbial polysaccharide supplied by Kelco International Limited as K1A112 (Industrial Grade) produced by microbial fermentation. |
| WELAN GUM : | A high molecular weight anionic heteropolysaccharide produced by microbial fermentation, and supplied by Kelco International Limited as K1A96 (Industrial Grade) |
| GUM TRAGACANTH : | Obtained from Astragalus gummifer or other Asiatic species of Astragalus (Leguminosae family) as a dried, gummy exudate, and supplied by Arthur Branwell & Co. Limited as LUXURA 3414. |
| SODIUM ALGINATE : | an extract from brown seaweeds ; supplied by Arthur Branwell & Co. Limited as LUXURA 3418. |
| GUAR GUM : | obtained from ground endosperms of Cyanopsis tetragonoloba ; supplied by Arthur Branwell & Co. Limited as LUXURA 3417. |
| MAIZE STARCH : | Supplied by Arthur Branwell & Co. Limited as LUXURA 3422. |
| CAB-O-SIL : | A fumed silica, grade 175, supplied by Wacker Chemicals Ltd. |
| KOLLIDON 90 : | Polyvinyl pyrrolidone supplied by BASF. |
| POLYOX : | poly (ethylene oxide), grade WSR-301, supplied by Union Carbide (UK) Ltd. |
| CELACOL : | Hydroxy propyl methyl cellulose, grade HPM 450 DS, supplied by Courtaulds Chemicals and Plastics. |
| NATROSOL : | Hydroxyethyl cellulose, grade 250 HBR, supplied by Hercules BV. |
| CARBOPOL : | Carboxypolymethylene, grade 940, supplied by B. F. Goodrich. |

**EXAMPLE 1**

Tests such as those described in this Example may be used to investigate the suitability of specific sulphur dioxide-liberating agent/moderator combinations for providing extended release of sulphur dioxide.

6

The sulphur dioxide-liberating compound was dissolved in 100 ml of water or in 100 ml of a 0.1 M aqueous solution of the moderator, as appropriate, in a 500 ml vessel fitted with a perforated top and maintained at about 18°C, to give solutions of the concentrations indicated in Table 1 below (as percentage concentration weight: volume). The initial pH of the solution was measured and then the sulphur dioxide concentration was measured within the vessel by means of Draeger tubes after set intervals ranging from 1/4 hour to 12 days. (The perforated top prevented complete accumulation of sulphur dioxide, which would not give useful results for the present purposes ; the leakage of sulphur dioxide through the perforated top was greater than the leakage that there would be from a "trap-top" disposal unit.) The results of these tests are summarised in Table 1.

EP 0 431 844 A2

TABLE 1

| Test | SO$_2$-liberating compound | | Moderator | pH of final solution | SO$_2$ concentration (p.p.m.) | | | | |
|---|---|---|---|---|---|---|---|---|---|
| | Type | Conc. % wt/vol | | | 1/4h | 1½h | 18h | 7h | 12d |
| 1 | sodium metabisulphite | 5 | none | 4.3 | 50 | 70 | 140 | 48 | 9 |
| 2. | sodium metabisulphite | 5 | thiosulphate (5% wt/vol.) | 4.4 | 10 | 28 | 40 | 6 | 5 |
| 3 | sodium metabisulphite | 5 | thiosulphate/phosphate | 4.7 | 16 | 18 | 18 | 18 | 16 |
| 4 | sodium dithionite | 5 | none | 6.0 | 180 | — | 200 | 0 | 0 |
| 5. | sodium dithionite | 5 | triethylamine (pH 11.4) | 10.4 | 120 | — | 110 | 20 | 14 |
| 6 | sodium dithionite | 5 | boric acid-borate (pH 9.2) | 7.2 | 140 | — | — | 20 | .12 |
| 7 | sodium dithionite | 2.5 | boric acid-borate (pH 9.2) | 8.4 | 52 | — | 56 | 15 | 10 |
| 8 | none | — | thiosulphate (5% wt/vol.) | 5.3 | No SO$_2$ liberated | | | | |

From the results given in Table I it can be seen that the presence of the moderator in the compositions according to the invention (tests 2, 3, 5, 6 and 7) gives extended release of sulphur dioxide, as compared with the release of sulphur dioxide from the sulphur-dioxide-liberating compounds without a moderator (tests 1 and 4), thus enabling an active concentration of sulphur dioxide to be maintained above the solution for an extended period of time. Test 8 simply shows that sodium thiosulphate does not act as a sulphur dioxide-liberating compound. Test 3 is particularly notable in that the rate of sulphur dioxide liberation was such that a practically constant sulphur dioxide concentration was maintained for the whole period of the test, and in that the sulphur dioxide concentration was still above 10 p.p.m. after 3 weeks. In tests 5, 6 and 7, the sulphur dioxide concentration was maintained at an active level throughout the test, whereas sulphur dioxide production from the sodium dithionite alone (test 4) has ceased after 5 days.

## EXAMPLE 2

A number of thickening agents were tested for suitability for use in accordance with the invention using formulation A below :

### Formulation A

|  | % by weight |
|---|---|
| sodium metabisulphite | 67.9 |
| sodium thiosulphate | 11.5 |
| potassium dihydrogen orthophosphate | 17.5 |
| perfume, flow agent | 3.1 |
|  | 100.0 |

It was found that the minimum volume of water that would completely dissolve 80 g of formulation A was 100 ml. The solution obtained had a pH of approximately 4.

The thickening agent to be tested was added to the quantity of water indicated in Table 2 and mixed with a high shear Silverson mixer (Model L2R) until a homogeneous viscous solution was formed.

80 g of Formulation A were added gradually to each viscous solution produced as indicated above and the mixture was stirred until the constituents of Formulation A had completely dissolved. The viscosity of the preparation obtained was observed immediately, and, where appropriate, after an interval of two weeks during which sulphur dioxide was liberated from the preparation.

Table 2 indicates the results of tests on a number of thickening agents :

TABLE 2

| Prep. | Thickening agent | vol of water (ml) | concentration of thickening agent in preparation (% m/m) | pH of preparation | Comments. |
|---|---|---|---|---|---|
| 1 | Kelzan (industrial grade) | 100 | 1 | 3.9 | Preparation is a uniform viscous solution. Viscosity of preparation increases with time. |
| 2 | Kelzan (industrial grade) | 200 | 1 | 4.1 | Uniform viscous solution of the same consistency as in preparation 1. Viscosity increases with time. |

EP 0 431 844 A2

TABLE 2 (continued)

| Prep. | Thickening agent | vol of water (ml) | concentration of thickening agent in preparation (% m/m) | pH of preparation | Comments. |
|---|---|---|---|---|---|
| 3 | Kelzan (industrial grade) | 100 | 2 | 4.0 | Solution slightly more viscous than in preparation 1, but will still flow readily. Viscosity increases with time. |
| 4 | Kelzan (industrial grade) | 200 | 2 | 4.1 | As Test 3 |

EP 0 431 844 A2

## TABLE 2 (continued)

| Prep. | Thickening agent | vol of water (ml) | concentration of thickening agent in preparation (% m/m) | pH of preparation | Comments. |
|---|---|---|---|---|---|
| 5 | Rhamsan Gum | 100 | 0.5 | 3.9 | Thickening agent agglomerates if not dispersed quickly. Preparation is viscous solution. No significant decrease in viscosity with time. |
| 6 | Welan Gum | 100 | 0.5 | 3.9 | As Test 5 |

TABLE 2 (continued)

| Prep. | Thickening agent | vol of water (ml) | concentration of thickening agent in preparation (% m/m) | pH of preparation | Comments. |
|---|---|---|---|---|---|
| 7 | Gum tragacanth | 100 | 2 | 4.1 | Thickening agent readily disperses and thickens. No decrease in viscosity on addition of formulation A. No significant decrease in viscosity of preparation with time. |
| 8 | Sodium alginate | 100 | 1 | 4.0 | As Test 7. |

EP 0 431 844 A2

TABLE 2 (continued)

| Prep. | Thickening agent | vol of water (ml) | concentration of thickening agent in preparation (% m/m) | pH of preparation | Comments. |
|---|---|---|---|---|---|
| 9 | Guar gum | 100 | 0.5 | 4.0 | Thickening agent tends to agglomerate if not mixed carefully. Preparation is viscous solution. No significant decrease in viscosity of preparation with time. |
| 10 | Maize starch | 100 | 4 | 4.1 | Thickening agent readily disperses and thickens. No decrease in viscosity on addition of formulation A, but viscosity of preparation decreased within two weeks, with precipitation of starch. |

## TABLE 2 (continued)

| Prep. | Thickening agent | vol of water (ml) | concentration of thickening agent in preparation (% m/m) | pH of preparation | Comments. |
|-------|------------------|-------------------|----------------------------------------------------------|-------------------|-----------|
| 11 | Cab-o-sil | 100 | 10 | 4.0 | Viscous solution with consistency similar to that of wall-paper paste. Preparation rapidly dries out. |
| 12 | Kollidon 90 | 100 | 1 | - | Addition of Formulation A causes precipitation of thickening agent from viscous aqueous solution. |

## TABLE 2 (continued)

| Prep. | Thickening agent | vol of water (ml) | concentration of thickening agent in preparation (% m/m) | pH of preparation | Comments. |
|---|---|---|---|---|---|
| 13 | Polyox | 100 | 1 | – | As preparation 12 |
| 14 | Celacol | 100 | 5 | – | As preparation 12 |
| 15 | Natrosol | 100 | 2 | – | As preparation 12 |
| 16 | Carbopol | 100 | 2 | – | As preparation 12 |

EP 0 431 844 A2

EP 0 431 844 A2

Each of preparations 1 to 9 had a viscosity of at least 10 Pa.s for at least two weeks and is a preparation in accordance with the invention. Preparations 10 to 16 are not stable viscous solutions, and are not preparations in accordance with the invention.

**EXAMPLE 3**

Preparations 17 and 18 having the composition indicated below were prepared using the method indicated in Example 2 :

## Preparation 17

|  | % by weight |
|---|---|
| sodium metabisulphite | 29.8 |
| triethylamine | 12.8 |
| Kelzan | 1.0 |
| water | 56.4 |
|  | 100.0 |

## Preparation 18

| sodium dithionite | 29.8 |
|---|---|
| potassium dihydrogen orthophosphate | 7.7 |
| sodium thiosulphate | 5.1 |
| Kelzan | 1.0 |
| water | 56.4 |
|  | 100.0 |

In each case a viscous solution (a smooth homogeneous paste) having a viscosity of more than 10 Pa.s was obtained, and after two weeks no decrease in viscosity was observed, indicating that the preparation was a stable viscous solution.

### Example 4

The sulphur dioxide emission of Preparations 1 to 9 in Example 2 and Preparations 17 and 18 in Example 3 was tested as indicated below.

A desired quantity of the preparation was placed in a plastic container of size 16.0 × 2.7 × 3.5 cm and hung inside a 30 litre "trap-top" container, about 15 cm from the top. The sulphur dioxide concentration inside the "trap-top" container was measured by means of a portable Gastec sulphur dioxide monitor (Model SD-1050). The "trap-top" containers were stored at room temperature, which varied from about 20°C to 25°C. The results obtained are shown in Table 3. In each case, the values given for sulphur dioxide emission are based on the average of the readings from three identical trap-top containers.

17

## TABLE 3

| Preparation tested | Weight of preparation used (g) | SO$_2$ concentration (ppm) |
|---|---|---|
| 1 | 50 | 50 to 200 for 6 weeks |
| 1 | 100 | 100 to 300 for 6 weeks |
| 1 | 180 | 100 to 300 for 6 weeks |
| 2 | 50 | 30 to 200 for 6 weeks |
| 2 | 100 | 50 to 300 for 6 weeks |
| 2 | 180 | 100 to 300 for 6 weeks |
| 3 | 180 | 100 to 300 for 6 weeks |
| 4 | 180 | 100 to 300 for 6 weeks |
| 5 | 100 | 100 to 300 for 6 weeks |
| 6 | 100 | 100 to 300 for 6 weeks |
| 7 | 100 | 100 to 300 for 6 weeks |
| 8 | 100 | 100 to 300 for 6 weeks |
| 9 | 100 | 100 to 300 for 6 weeks |
| 17 | 100 | 20 to 240 for 6 weeks |
| 18 | 100 | 50 to 190 for 6 weeks |

### EXAMPLE 5

A number the of preparations indicated in Example 2 were evaluated for bactericidal efficacy.

A desired quantity of each preparation was placed in a plastic container as specified in Example 4 and hung inside a 30 litre "trap-top" container, about 15 cm from the top. The trap-top containers were placed inside ladies' toilet cubicles and used normally. The SO$_2$ level was measured weekly using a portable Gastec SO$_2$ monitor (Model SD-1050). After six weeks a Simplicity No. 1 sanitary towel, artificially soiled with an overnight culture of E.coli, was introduced into each container. After 24 hours, the artificially soiled towel was removed and the number of bacteria surviving measured. The results are shown in Table 4, in which the preparations used are identified by the preparation numbers given in Table 2. The results show that the SO$_2$ liberated after an extended time of six weeks is sufficient to cause sterilization of the contents of a "trap-top" container.

## TABLE 4

| Prep | Weight of paste per container (g) | Micro-organisms added per towel (expressed as $\log_{10}$) | Average number of micro-organisms recovered – detection limit = $10^3$ (expressed as $\log_{10}$) | Reduction in number of micro-organisms (expressed as $\log_{10}$) | Range of $SO_2$ concentration (ppm) |
|---|---|---|---|---|---|
| 1 | 180 | 9.51 | $< 3$ | $> 6.51$ | 41 – 354 |
| 1 | 100 | 9.51 | $< 3$ | $> 6.51$ | 58 – 301 |
| 2 | 100 | 9.51 | $< 3$ | $> 6.51$ | 50 – 81 |
| 1 + 20 ml glyc- erol | 100 | 9.16 | $< 3$ | $> 6.16$ | 72 – 174 |
| 7 | 100 | 9.16 | 3.50 | 5.66 | 43 – 206 |

EP 0 431 844 A2

## Example 6

This example shows the effect of articles to be sterilized on the concentration of sulphur dioxide when using two of the preparations (one in a modified form) specified in Example 2.

100 g of each preparation was placed in a plastic container as specified in Example 4 and hung inside a respective 30 litre "trap-top" container, 15 cm from the top. After 15 days at room temperature the sulphur dioxide concentration in each trap-top container was measured, using a portable Gastec sulphur dioxide monitor (Model SD-1050), before and after the addition of a Simplicity No. 1 sanitary towel loaded with 5 ml horse blood. The results are given in Table 5, in which the preparations used are identified by the preparation numbers given in Table 2. Each sulphur dioxide concentration given in Table 5 is the average of the readings from three identical trap-top containers.

### TABLE 5

### Sulphur dioxide concentration (ppm)

| Time (hours) | Preparation 8 | Preparation 9 (modified to include 1% m/m guar gum) |
|---|---|---|
| 0 (before addition of article to be sterilized) | 200 ± 33 | 227 ± 73 |
| 0.25 | 115 ± 20 | 99 ± 36 |
| 1 | 96 ± 20 | 73 ± 21 |
| 2 | 75 ± 29 | 72 ± 18 |
| 2.5 | 96 ± 9 | 72 ± 18 |
| 4 | 117 ± 16 | 83 ± 19 |
| 5 | 130 ± 11 | 86 ± 19 |
| 6 | 135 ± 11 | 93 ± 21 |
| 7 | 145 ± 11 | 97 ± 23 |
| 24 | 260 ± 22 | 193 ± 48 |

As shown in Table 5, the sulphur dioxide concentration drops after the addition of the article to be sterilized, but remains within the desired range. Within about 4 hours the sulphur dioxide level starts to increase again, and after 24 hours the concentration has recovered to a value similar to that present before the addition of the article to be sterilized. The results show that active levels of sulphur dioxide are maintained in the trap-top containers despite the addition of articles to be sterilized, and that the preparations of the invention enable sulphur dioxide concentrations to return to a desired higher level within a relatively short period.

EP 0 431 844 A2

## Claims

1. A composition which comprises a thickening agent and an agent capable of liberating a vapour-phase disinfectant on contact with water, the proportion by weight and nature of the thickening agent being such that, when the composition is mixed with the minimum amount of water required to dissolve the said agent, a stable viscous solution (as hereinbefore defined) is formed.

2. A composition capable of giving extended release of sulphur dioxide in the presence of moisture, which comprises an agent that will liberate sulphur dioxide on contact with water, a moderator for moderating the liberation of sulphur dioxide, and a thickening agent, the proportion by weight and nature of the thickening agent being such that, when the composition is mixed with the minimum amount of water required to dissolve the sulphur dioxide-liberating agent and the moderator, a stable viscous solution (as hereinbefore defined) is formed.

3. A composition as claimed in claim 1 or claim 2, in the form of an aqueous solution.

4. A preparation which comprises a thickening agent and an agent capable of liberating a vapour-phase disinfectant on contact with water, the preparation being in the form of a stable viscous solution (as hereinbefore defined).

5. A preparation capable of giving extended release of sulphur dioxide, which comprises an agent that will liberate sulphur dioxide on contact with water, a moderator for moderating the liberation of sulphur dioxide, a thickening agent, and water, the preparation being in the form of a stable viscous solution (as hereinbefore defined).

6. An invention as claimed in any one of claims 3 to 5, wherein the solution has viscosity of at least 10 Pa.s, preferably at least 20 Pa.s.

7. An invention as claimed in any one of claims 1 to 6, wherein the viscosity of the solution, immediately after preparation, does not exceed 200 Pa.s, and is advantageously in the range of from 10 to 100 Pa.s, preferably from 20 to 40 Pa.s.

8. An invention as claimed in any one of claims 3 to 5, wherein the solution, on release of vapour-phase disinfectant therefrom, retains a viscosity of at least 10 Pa.s for at least four weeks, preferably for at least six weeks.

9. An invention as claimed in any one of claims 1 to 8, wherein the thickening agent is such that it does not precipitate out of an aqueous solution having an ionic concentration up to 800 g/l and/or is such that it does not precipitate out of an aqueous solution having a pH of from 2 to 7.

10. An invention as claimed in any one of claims 1 to 9, wherein the preparation or an aqueous solution of the composition can retain for at least four weeks sufficient water to maintain the agent capable of liberating a vapour-phase disinfectant, or the sulphur dioxide-liberating compound and the moderator, in solution.

11. An invention as claimed in any one of claims 1 to 10, wherein the thickening agent is such that the preparation, or an aqueous solution of the composition, can exhibit pseudoplastic properties.

12. An invention as claimed in any one of claims 1 to 11, wherein the thickening agent is a high molecular weight polysaccharide.

13. An invention as claimed in any one of claims 1 to 11, wherein the thickening agent is a carbohydrate polymer of high molecular weight comprising acidic and/or neutral monosaccharide units joined by glycosidic bonds.

14. An invention as claimed in any one of claims 1 to 11, wherein the thickening agent is a high molecular weight anionic heteropolysaccharide.

15. An invention as claimed in any one of claims 1 to 11, wherein the thickening agent is a xanthan gum.

21

16. An invention as claimed in any one of claims 2, 3, 5, and 6 to 15, so far as they are dependent on claim 2 or claim 5, wherein the sulphur dioxide-liberating agent is a metabisulphite or a dithionite.

17. An invention as claimed in any one of claims 2, 3, 5, and 6 to 16 so far as they are dependent on claim 2 or claim 5, wherein the moderator is selected from thiosulphates, hydroquinones, and buffers, the buffers preferably being selected from phosphates, borates and amines.

18. An invention as claimed in any one of claims 2, 3, 5 and 6 to 15 so far as they are dependent on claim 2 or claim 5, which comprises a metabisulphite as the sulphur dioxide-liberating agent, and a thiosulphate and a phosphate as moderators.

19. A sealed container containing a composition as claimed in claim 3 or a preparation as claimed in any one of claims 4 to 18, the container preferably having a removable closure member.

20. A disposal unit for articles requiring sterilizing, which comprises a closed receptacle containing a quantity of a composition as claimed in claim 3 or a preparation as claimed in any one of claims 4 to 18.

21. A method of sterilising an article, especially a soiled sanitary or surgical dressing or a disposable diaper, which comprises bringing the article into contact with or into the proximity of a composition as claimed in claim 3 or a preparation as claimed in any one of claims 4 to 18, the article preferably being brought into contact with or into the proximity of the composition or preparation by being placed into a closed receptacle, preferably a "trap-top" container, containing a quantity of the composition or preparation.